# EUROPEAN PATENT SPECIFICATION

(11) **EP 2 618 752 B1**
(45) Date of publication and mention of the grant of the patent: **28.04.2021**
(21) Application number: 11761788.6
(22) Date of filing: 20.09.2011
(51) Int. Cl.: A61B 17/29, A61B 17/00

(54) **LAPAROSCOPIC INSTRUMENT WITH ATTACHABLE END EFFECTOR**
LAPAROSKOPISCHES INSTRUMENT MIT BEFESTIGBAREM GREIFORGAN
INSTRUMENT LAPAROSCOPIQUE ÉQUIPÉ D'UN ORGANE EFFECTEUR AMOVIBLE

(30) Priority: 24.09.2010 US 889458; 24.09.2010 US 889454
(43) Date of publication of application: 31.07.2013
(73) Proprietor: Ethicon Endo-Surgery, Inc., Cincinnati, OH 45242 (US)
(72) Inventor: NOBIS, Rudolph, H., Mason, OH 45040 (US); SPIVEY, James, T., Cincinnati, OH 45226 (US); HESS, Christopher, J., Cincinnati, OH 45206 (US); HUEY, Kevin, M., Cincinnati, OH 45202 (US)
(74) Representative: Tunstall, Christopher Stephen
(86) International application number: PCT/US2011/052327
(87) International publication number: WO 2012/040183

(56) References cited:
- JP-A- 2005 261 734
- US-A1- 2008 004 656
- US-A1- 2011 087 267

## Description

### BACKGROUND

The present disclosure relates in general to surgical devices and procedures, and more particularly to minimally invasive surgery.

Surgical procedures are often used to treat and cure a wide range of diseases, conditions, and injuries. Surgery often requires access to internal tissue through open surgical procedures or endoscopic surgical procedures. The term "endoscopic" refers to all types of minimally invasive surgical procedures including laparoscopic, arthroscopic, natural orifice intraluminal, and natural orifice transluminal procedures. Endoscopic surgery has numerous advantages compared to traditional open surgical procedures, including reduced trauma, faster recovery, reduced risk of infection, and reduced scarring. Endoscopic surgery is often performed with an insufflatory fluid present within the body cavity, such as carbon dioxide or saline, to provide adequate space to perform the intended surgical procedures. The insufflated cavity is generally under pressure and is sometimes referred to as being in a state of pneumoperitoneum. Surgical access devices are often used to facilitate surgical manipulation of internal tissue while maintaining pneumoperitoneum. For example, trocars are often used to provide a port through which endoscopic surgical instruments are passed. Trocars generally have an instrument seal, which prevents the insufflatory fluid from escaping while an instrument is positioned in the trocar.

US 2008/0004656 describes and illustrates a surgical forceps primarily for use in performing endoscopic or arthroscopic surgery. The surgical instrument includes a shaft disposed at least partially in a housing. The housing is tubular and includes a proximal end and a distal end. A fixed handle and a movable handle are connected to the proximal end of the shaft. As the movable handle is actuated, it longitudinally reciprocates the shaft within the housing. The surgical instrument includes a detachable tool assembly that can be connected to the shaft. The detachable tool assembly is separable from the shaft and the housing to allow the user to change the tool. The tool assembly includes a clevis component. The proximal end of the clevis component is detachably coupled to the distal end of the housing. The distal end of the housing defines two slits extending inward to separate a pair of legs. The tool assembly also includes a connection rod. The proximal end of the rod defines a ball shape. Jaws of the tool assembly are movable in response to reciprocating movement of the connection rod. The surgical instrument includes a collet for detachably coupling the connection rod to the shaft. The collet is disposed within the housing. The collet includes a base and a plurality of expandable fingers extending from the base. The base of the collet is attached to the distal end of the shaft. Each finger of the collet includes a stem extending from the base and a tip disposed atop of the stem. The tips of the fingers flare apart from one another when not compressed. However, when compressed, the tips come together to form a hoop and the ball-shaped proximal end of the connection rod is captured between the base and the tips of the fingers. The housing defines a central opening and a cavity. The cavity is disposed adjacent the distal end of the housing. The cavity has a cross-sectional width greater than the cross-sectional width of the central opening. Since the base of the collet is attached to the distal end of the shaft, the collet moves within the housing as the shaft reciprocates based on motion of the movable handle. The collet is movable between an ejected position and operating positions. In the operating positions, the fingers of the collet are substantially disposed within the central opening, such that the fingers are collapsed to capture the ball-shaped proximal end of the connection rod. In the ejected position, the fingers of the collet are substantially disposed within the cavity so that the fingers flare away from one another in the cavity to release the connection rod. In addition to the coupling provided by the collet and the connection rod, a secondary coupling is provided by the clevis component and the housing. Specifically, the proximal end of the clevis component is removably coupled to the distal end of the housing. The proximal end of the clevis component defines a recessed outer surface with a groove indented into the recessed outer surface. The distal end of the housing defines an inner surface with a tongue protruding inward. The tongue of the housing and the groove of the clevis component mate to couple the housing and clevis component together. The slits allow the distal end of the housing to expand, thus allowing connection and disconnection of the clevis component from the housing.

While surgical access devices are known, no one has previously made or used the surgical devices in accordance with the present invention.

### SUMMARY

The invention relates to a surgical device as defined in claim 1. Preferred embodiments are defined in the dependent claims.

A surgical device is provided that comprises an elongate shaft defining a longitudinal axis, the shaft comprising a distal end and a proximal end. There is a first arm on the elongate shaft comprising a lateral surface having a mating feature, the first arm being medially deflectable. A second arm comprises a lateral surface having a mating feature longitudinally spaced from the first arm, the second arm being axially slideable relative to the elongate shaft and being medially deflectable. An elongate pin is positioned medially relative to the second arm, the elongate pin being axially slideable relative to the first arm and second arm between a locked position preventing medial deflection of the first arm and second arm and an unlocked position allowing medial deflection of the first arm and second arm. The surgical device further comprises a surgical end effector selectively attachable and detachable to the mating feature of the second arm.

The surgical device may further comprise a lateral notch on the distal end of the first arm and a mating feature on the surgical end effector. The surgical device may have a mating feature comprised of a ring dimensioned to mate with the first arm lateral notch and may further have two or more second arms circumscribing the elongate pin. The surgical device may have two or more first arms and an opening defined by the two or more first arms and an elongate pin having an obturator tip. The surgical device may further comprise a handle operatively connected to the proximal end of the elongate shaft, the handle comprising a trigger controlling the axial movement of the second arm and an actuator controlling the axial movement of the elongate pin, wherein the actuator may be lockable. The end effector may have opposable members that move between open and closed positions in response to axial motion of the second arm.

### BRIEF DESCRIPTION OF DRAWINGS

While the specification concludes with claims which particularly point out and distinctly claim the invention, it is believed the invention will be better understood from the following description taken in conjunction with the accompanying drawings illustrating some non-limiting examples of the invention. Unless otherwise indicated, the figures are not necessarily drawn to scale, but rather to illustrate the principles of the invention.
Fig. 1 depicts surgical procedure with an instrument and loader holding an end effector;

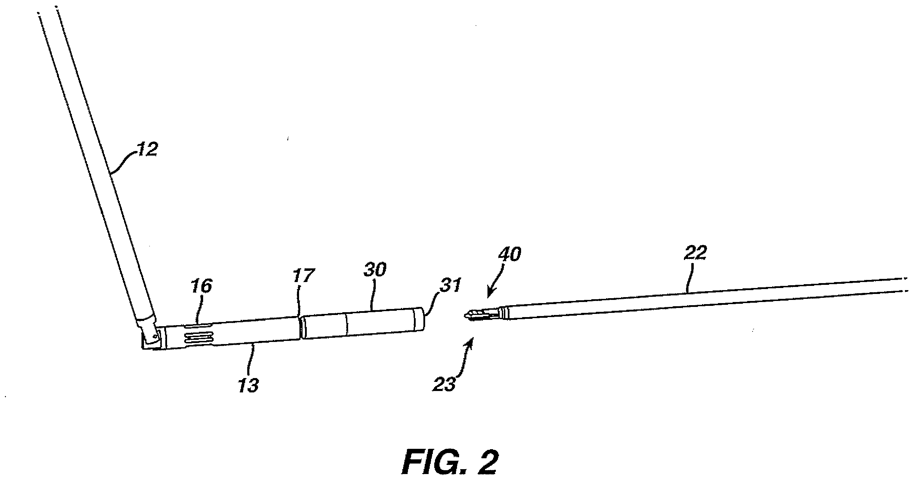

Fig. 2 depicts a close-up view of the distal ends of the instrument and loader in Fig. 1;
Fig. 3 depicts an instrument being inserted into an end effector;
Fig. 3A depicts an isometric cross-sectional view of an end effector;
Fig. 3B depicts an isometric cross-sectional view of an instrument partially inserted into an end effector;
Fig. 3C depicts an end effector with torque arms provided in the lateral surface of the end effector;
Fig. 3D depicts a close up of the end effector of Fig. 3C;
Fig. 3E depicts a cross section of the Fig. 3D end effector with an instrument inserted in the end effector;
Fig. 4 depicts an instrument attached to an end effector being withdrawn from a loader;
Fig. 4A depicts a loader with removable distal end;
Fig. 5 depicts an isometric close-up view of the distal end of an instrument in a locked position;
Fig. 6 depicts an isometric close-up view of the distal end of an instrument in an unlocked position;
Fig. 7 depicts an isometric cross-sectional view of the distal end of an instrument attached to an end effector;
Fig. 7A depicts an isometric cross-sectional view of the distal end of an instrument attached to an end effector with the pin advanced distally;
Fig. 8 depicts an isometric cross-sectional view of the distal end of an instrument attached to an end effector in a pushed-off configuration;
Fig. 9 depicts an instrument handle;
Fig. 10 depicts a bi-polar jawed end effector;
Fig. 11 depicts a cutting shears end effector;
Fig. 12 depicts a Maryland dissector end effector; and
Fig. 13 depicts an ultrasonic shears end effector;

### DETAILED DESCRIPTION

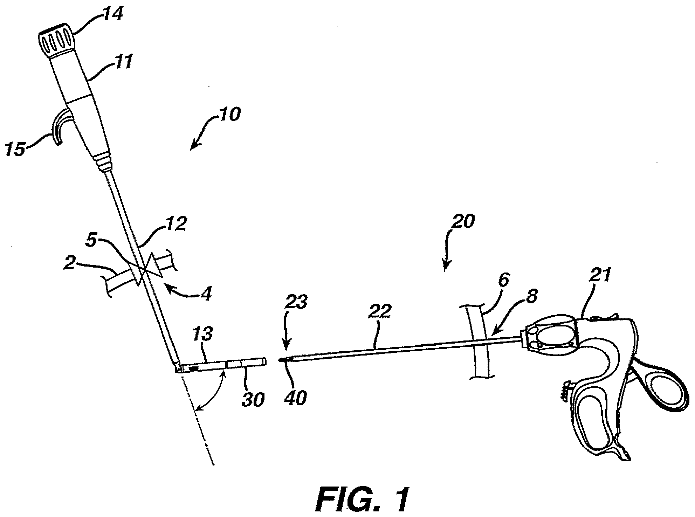

As shown in Fig. 1, instrument (20) comprises an elongate shaft (22) passing through an incision (8) of a tissue wall (6). A loader (10) comprises an elongate shaft (12) passing through an incision (4) of a tissue wall (2). The surgical end effector (30) is selectively attachable in vivo and detachable in vivo to the attachment mechanism (40) located at the distal end (23) of the instrument (20). In this example, the end effector is a jawed tissue grasper, but a variety of other end effectors could be also be used. The end effector (30) may be loaded ex vivo into the distal end (13) of the shaft (12), and then introduced into the surgical field through the incision (4). The loader (10) holds the end effector (30) during the in vivo attachment to and in vivo detachment from the instrument (20). The loader (10) and instrument (20) each includes ex vivo handles (11, 21) attached to the proximal ends of the shafts (12, 22) that enable surgeons to use the devices.

The tissue wall (2, 6) anatomies will vary based on the surgical procedure, but some non-limiting examples include percutaneous incisions into the abdomen, thorax, or pelvis. The incisions (4, 8) may be created with a cutting or puncturing instrument, and will typically be spaced from one another. The tissue walls (2, 6) may be the same or different anatomies. For instance, tissue walls (2, 6) may both be the abdominal wall. In another example, tissue wall (2) could be an organ (e.g., stomach, colon, esophagus, etc.) accessed through a natural orifice, while the incision (8) in tissue wall (6) could be percutaneous. In yet another example, incision (4) may provide access to the abdomen, while the incision (8) may provide access to the pelvis. If pneumoperitoneum is desired, the incisions may include instrument seals, such as those commonly found in trocars. In this example, the instrument seal (5) is schematically shown in incision (4) with the loader (10) passing through the seal (5), while the shaft (22) seals directly with the tissue wall (6) by virtue of the resilience of the tissue without the aid of a sealing device.

The loader shaft (12) in this embodiment is rigid and straight, but the shaft (12) could be curved or flexible, which would be beneficial for natural orifice transluminal introduction of the distal end (13) to the surgical field. The loader (10) may include an articulating distal end (13) controlled by the knob (14). The distal end (13) will typically be introduced and removed through the incision (4) in-line with the shaft (12), and then articulated in vivo to facilitate alignment between the end effector (30) and the shaft (22). The arm (15) is rigidly connected the handle (11) to facilitate grasping of the handle and rotational orientation of the articulated distal end (13) about the shaft (12) axis. In this embodiment, the distal end (13) of the loader (10) comprises a tube opening at the distal tip (17). The tube is dimensioned to receive the end effector (32). The tube (30) includes an engagement feature (16) for holding the end effector (32). While the engagement feature (16) may vary, in this embodiment a plurality of leaf springs provide an interference fit with the end effector (30) to frictionally hold the end effector in the tube. In this embodiment, when the end effector (30) is loaded in the tube, the distal end (32) is positioned in the tube and the proximal end (31) extends from the tube opening (17). This arrangement prevents the jaws of the end effector from opening. After the distal end (23) of the instrument (20) is attached to the proximal end (31) of the end effector (30), the end effector (3) can be pulled from the distal end (13) of the loader (10).

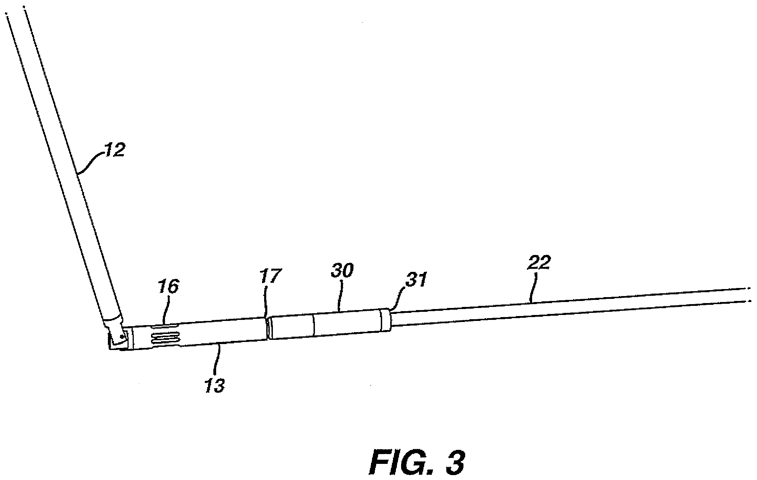

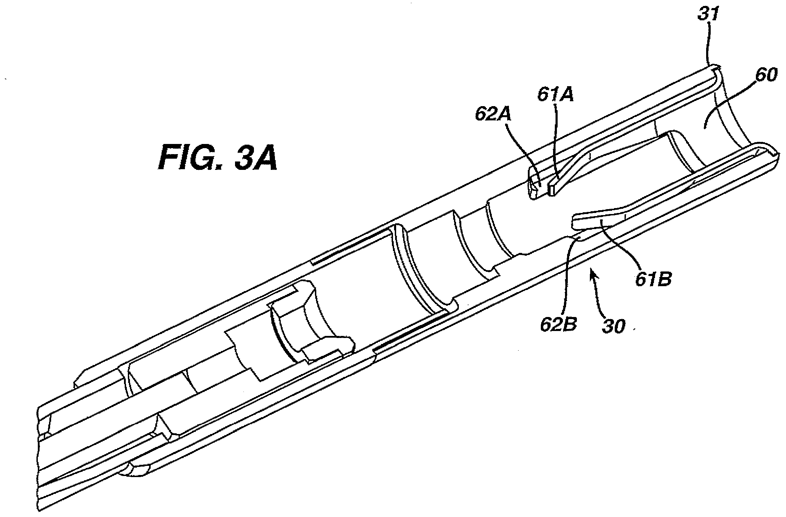

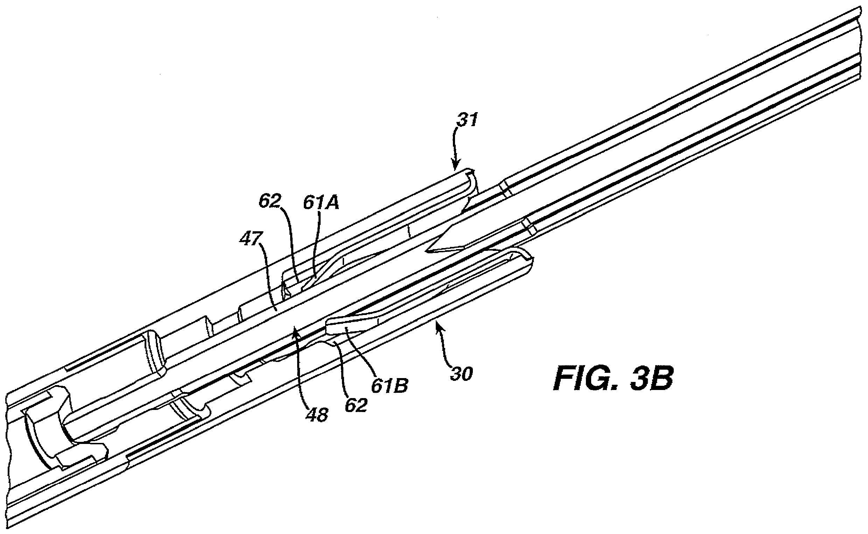

Fig. 3A depicts an example of an end effector provided with a torque key (60). The torque key, in one expression, is fixedly attached to proximal end (31) of end effector (30). Torque key (60) is provided with torque arms (61A, 61B). Torque arms (61) may be provided with a medial angular bend. End effector (30) may also be provided with torque arm recesses (62A, 62B) that permit the torque arms (61) to laterally deflect creating a variable inner diameter of end effector (30). Fig. 3B depicts the instrument shaft (22) partially inserted into end effector (30). In this depiction, torque arms (61) are aligned with flat surfaces on the shaft arms (47) and protrude medially into an opening (48) between shaft arms (47). When shaft (22) is inserted into end effector (30) and the torque arms (61) are not aligned with opening (48), they will remain deflected medially in recess (62) until the shaft (22) is rotated to align torque arms (62) with opening (48). When aligned with the opening (48), torque arms (61) permit transfer of rotational force from the shaft to the end effector.

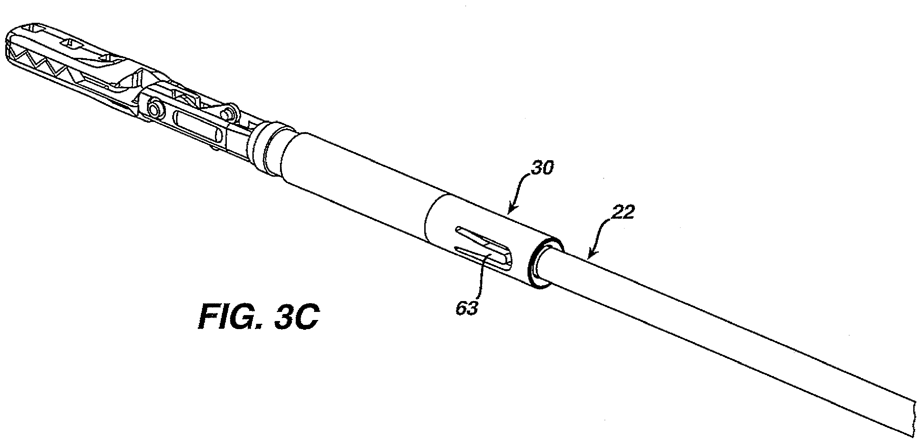

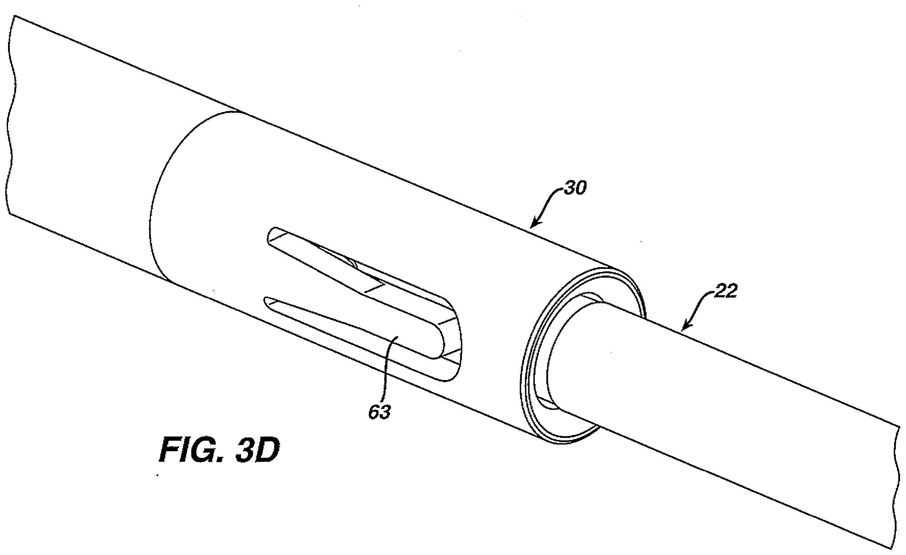

Figs. 3C and 3D depict another expression of the end effector (30). The proximal end of the end effector (30) is provided with flexible torque arm (63) formed from the lateral surface of end effector (30). When shaft (22) is inserted into end effector (30), torque arm (63) may deflect laterally where the opening (48) is not aligned with torque arm (63). To facilitate engagement with shaft (22) torque arm (63) may be provided with a chamfered surface. Upon rotation of the shaft (22), the torque arm will align with opening (48). When aligned with the opening (48), torque arm (63) permits transfer of rotational force from the shaft (22) to the end effector (30).

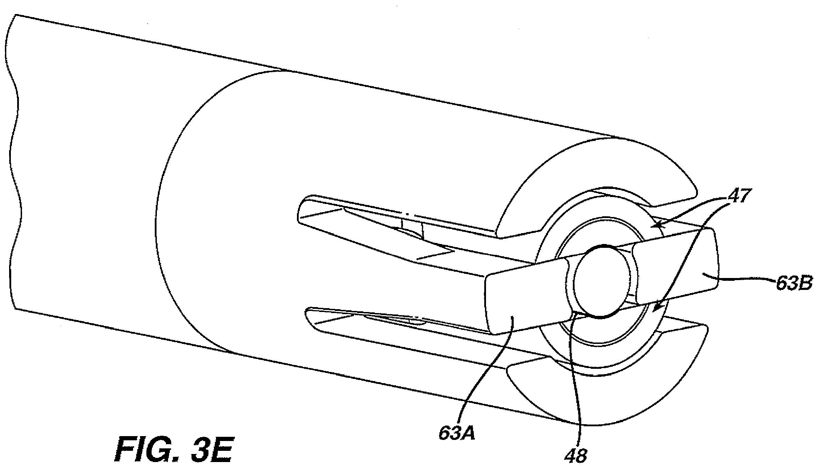

Fig. 3E depicts a cross sectional view of a shaft (22) inserted into end effector (30). In this expression, end effector (30) is provided with two torque arms (63A, 63B). Torque arms (63) are aligned to opening (48) defined by shaft arms (47) creating an interference fit.

In another expression of the surgical instrument, the torque arms (63) may be provided with recessed inner portions that mate with projections on the lateral surface of the shaft (not shown). The shaft projections may be flexible to facilitate entry of the shaft into the end effector. In yet another expression, the end effector may be provided with recesses (not shown) located on the medial surface of the end effector that mate with the projections on the lateral surface of the shaft.

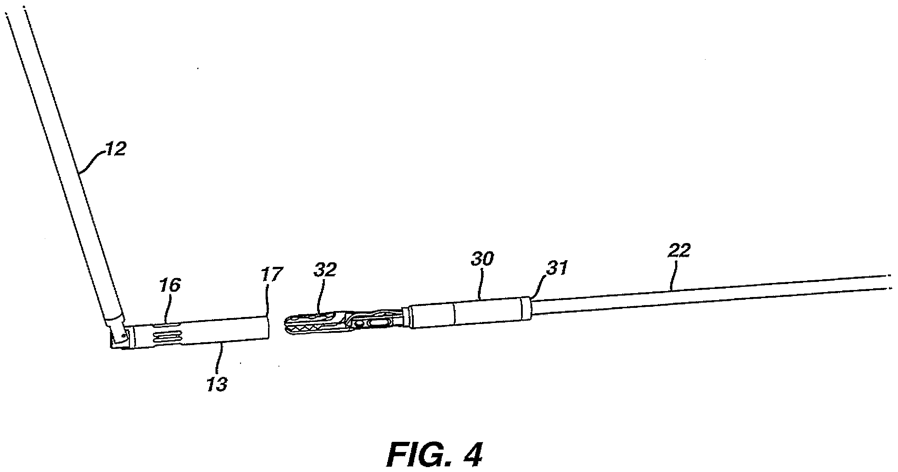

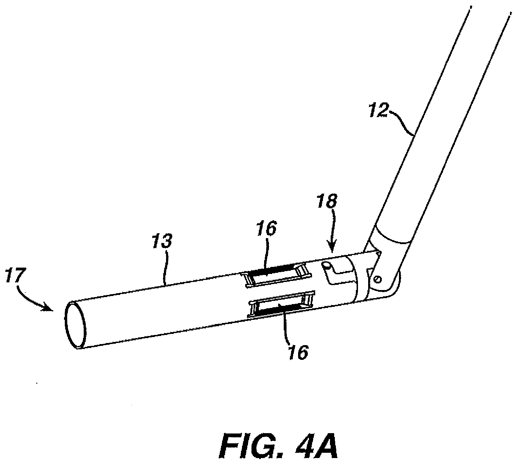

Fig. 4 depicts an instrument (20) attached to an end effector (32) being withdrawn from a loader (13). Fig. 4A depicts an alternative embodiment of a loader (10) where the distal end (13) is selectively attachable and detachable to the shaft (12). As shown in this example, this feature is enabled with a bayonet connection (18), but other connections are also contemplated including snap connections, threaded connections, and the like. One advantage of this alternative embodiment is that different distal end (13) configurations may be used to hold end effectors that may not be accommodated by a single sized tube.

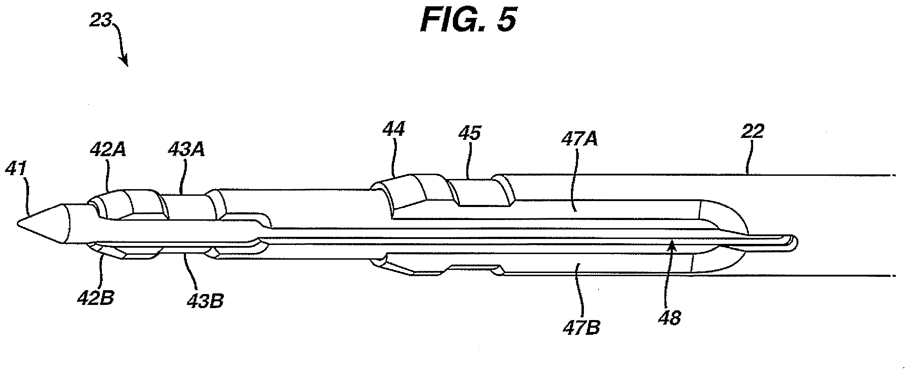

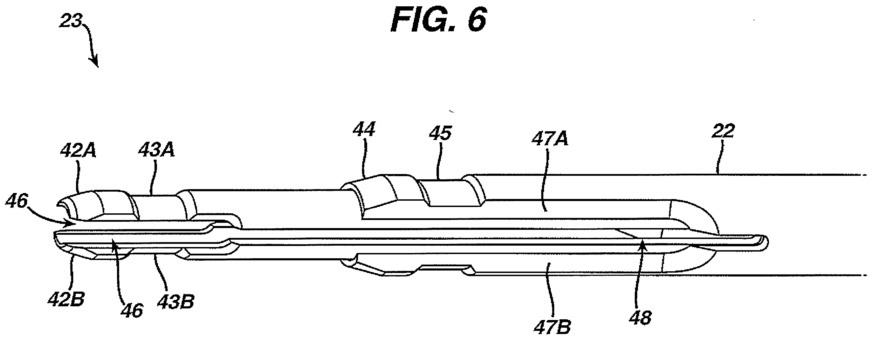

Figs. 5 and 6 depict a detailed view of one embodiment of an attachment mechanism (40) located at the distal end (23) of the shaft (22). The attachment mechanism (40) comprises a mating feature on the shaft (22), which in this embodiment is a circumferential groove (45) positioned on the lateral surface of shaft arms (47A, 47B). Shaft arms (47A, 47B) may be resiliently flexible into opening (48). The attachment mechanism (40) also comprises second arms (42A, 42B) projecting distally from the distal end (44) of the shaft (22). The second arms may be axially slideable relative the shaft (22) and are resiliently deflectable medially into the gap (46). The second arms each comprise a mating feature, which in this embodiment comprises a stepped lateral notch (43A, 43B). An elongate pin (41) is positioned medially relative the second arms (42) and shaft arms (47) and is axially slideable relative the second arms (42) and shaft arms (47) between a locked position preventing medial deflection of the arms (42 and 47) (an example of which is shown in Fig. 5) and an unlocked position allowing medial deflection of the arms (an example of which is shown in Fig. 6). The pin (41) and second arms (42) may each slide independently relative the shaft (22) and shaft arms (47). FIG 6 shows the pin (41) fully retracted inside shaft (22) allowing medial deflection of shaft arms (47).

As shown in the embodiment of Fig. 5, the elongate pin (41) may include a pointed obturator tip. In this configuration the distal end (23) may be used to puncture through the tissue wall (6). The distal ends of the second arms (42) and distal end (44) of the shaft arms (47A, 47B) include tapered surfaces to facilitate passing through the incision (8).

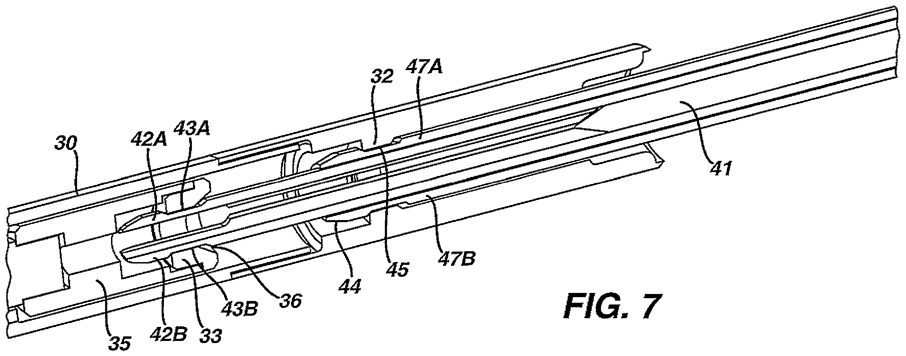

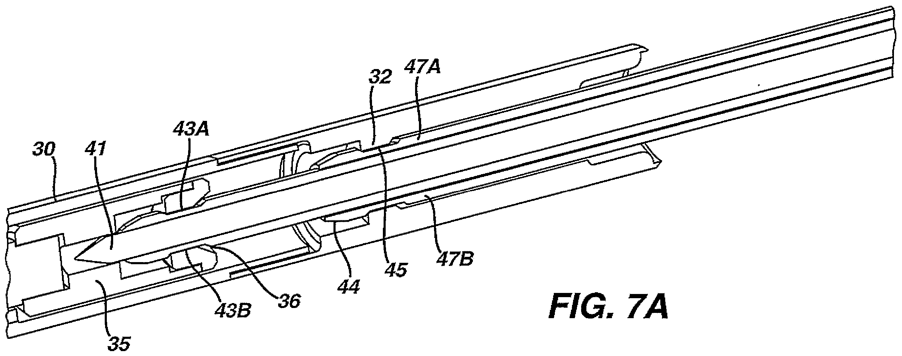

Fig. 7 shows the attachment mechanism (40) attached to the end effector (30). The groove (45) of the shaft arms (47) mates the rib (32) of the end effector (30) preventing relative axial motion. The lateral grooves (43A, 43B) of the second arms (42) mate the ring (33) of the end effector (30) preventing relative axial motion. The rib (32) is rigidly connected to the outer housing (37) of the end effector (30), and the ring (33) is rigidly connected to the jaw actuator (34) via the coupling (35). When the elongate pin (41) is fully advanced, medial deflection of the second arms (42) and the shaft arms (47) is inhibited (see Fig. 7A). Accordingly, axial movement of the arms (42) relative the shaft (22) will cause axial movement of the jaw actuator (34) relative the housing (37), thereby causing the jaws to open and close.

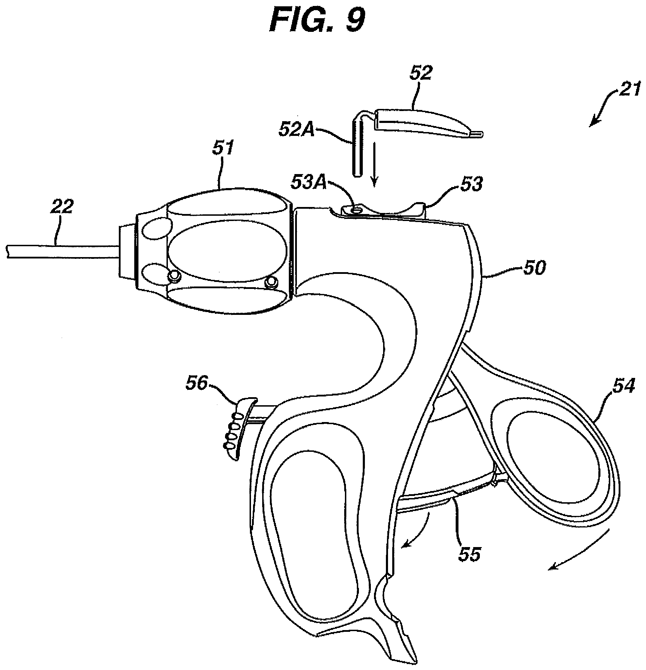

Fig. 9 shows and example of the handle (21) for the instrument (20). The handle (21) includes a base (50). A knob (51) rotates the attachment mechanism (40) about the axis of the shaft (22), which will also rotate an attached end effector (30). The trigger (54) pivots relative the base (50) causing axial movement of the second arms (42) and the pin (41) relative the shaft (22). Operation of the trigger (54) will operate the jaws on an attached end effector (30). The latch (55) pivots relative the base (50) between a locked position (as shown in figure) to prevent operation of the trigger (54) and an unlocked position recessed in the base (50). During seating with the end effector (30), the latch (55) may be locked to maintain the same relative axial spacing of the corresponding the mating features (43, 45) as the mating features (33, 32), resulting in resulting in a single "snap" feedback. The trigger lock (56) can lock/unlock the trigger in/from its depressed position. An actuator (53), which in this embodiment is a slider, controls axial movement of the pin (41) relative the second arms (42). The distal most position of the actuator (53) relative the base (as shown in the figure) places the pin (41) in its locked position, and the proximal most position places the pin (41) in its unlocked position. The pin lock (52) includes a pin (52A) which went inserted into the hole (53A) maintains the pin (41) and second arms (42) in the extended and locked positions as shown in Fig. 5.

The following describes one method for attaching the end effector (30) to the shaft (22). The distal end (23) is introduced in into the proximal end (31) of the end effector (30) with the pin (41) in the unlocked position. The shaft (22) deflects the torque arms (61) laterally into recesses (62) when the torque arms are not aligned with the opening (48). In another expression, torque arm (63) deflects laterally upon shaft (22) insertion into the end effector (30). When the torque arm (61, 63) are aligned with the opening (48), they do not deflect and rest adjacent to opening (48) on the lateral surfaces of shaft arms (47) permitting rotation of the end effector. As the arms (42) are advanced axially into the end effector (30), the chamfered lead (36) of the ring (33) medially deflects the arms (42) until the ring (33) is seated into the lateral notches (43). Simultaneously the shaft arms (47) advance axially into the end effector (30), and the tapered end (44) aligns the rib (32) to seat into the groove (45). In both cases, the surgeon may feel a tactile "click" indicating proper engagement. Once fully seated in the end effector (30), the pin (41) may be slid to the locked position thereby attaching the end effector (30) to the instrument (20). Once attached, the surgeon may pull the end effector from the loader (10), and the loader (10) may then be removed from the surgical field. When the end effector (30) is attached to the shaft (22) and the torque arm (61, 63) are not aligned with the opening (48), the surgeon may grip tissue or another instrument and rotate the knob (51) until the torque arms (61) seat in the opening (48). The surgeon may then manipulate tissue with the end effector (30) as needed for the surgical procedure.

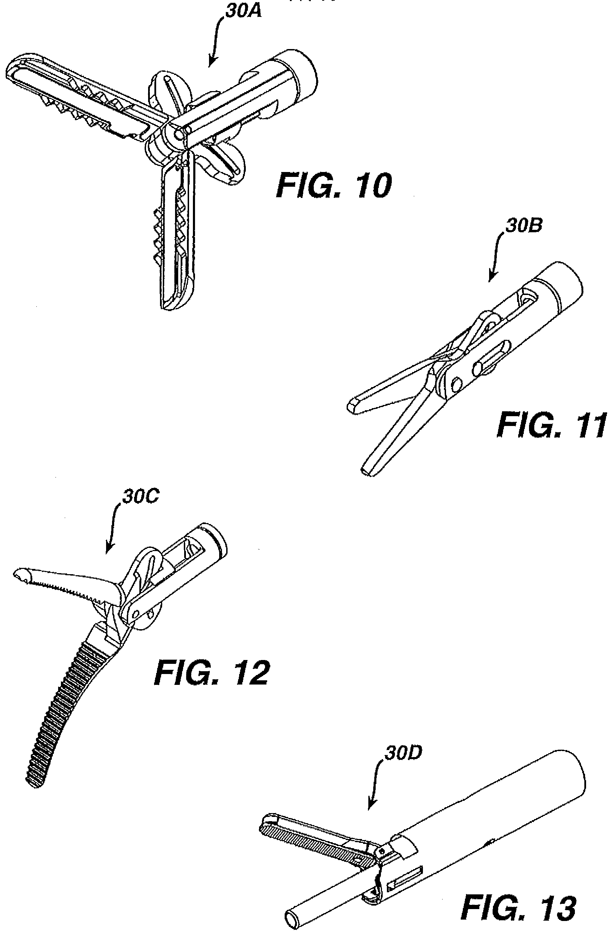

Figs. 10-13 illustrate some non-limiting examples of alternative end effectors (30A-D) that may attached to the distal end (23) of the instrument (20). In addition to the loader (10) and instrument (20), all or a portion of the end effectors (30, 30A, 30B, 30C, 30D) may be bundled as part of a kit so the surgeon may interchange the attached end effector as needed for a surgical procedure. All the end effectors examples shown here have cooperating jaws; however, non-jawed end effectors could also be employed such as hook knives, snares, and the like. In the case of end effectors that require energy, appropriate energy transmission mechanisms known in the art should be added to the handle (21) and shaft (22). For instance, appropriate electrical connections can be added for the bi-polar forceps end effector (30A). Similarly, an ultrasonic transducer and waveguide can be added for the ultrasonic shears end effector (30D).

The following describes one method for using the devices during a laparoscopic surgical procedure. An instrument (20) is obtained and passed through incision (8). The incision (8) may be a precutaneous incision formed at least partially by a puncture formed with the obturator on the pin (41) in the configuration shown in Fig. 5. The pin lock (52) and latch (55) may be secured to the slider (53) and trigger (54), respectively. After the puncture, the pin lock (52) may be removed.

A loader (10) and end effector (30) are obtained. The end effector (30) may be selected from a plurality of end effectors provided in a kit. The end effector (30) is loading ex vivo into the distal end (13) of the loader (10). The distal end (13) of the loader (10) with the loaded end effector (30) is passed through incision (4). The second incision (4) may also be percutaneous incision spaced from the first incision (8), and may include passing the distal end (13) with the loaded end effector (30) through a trocar. The distal end (13) may be articulated to facilitate orientation between the proximal end (31) of the end effector (30) and the attachment mechanism (40). The actuator (53) is slid proximally to move the pin (41) to its unlocked position. The distal end (23) of the instrument (20) is advanced into the proximal end (31) of the end effector (30) until the respective mating features of the instrument (20) and end effector (30) are engaged. The actuator (53) may then be slid distally thus advancing the pin (41) to its locked position. The end effector (30) has now been attached in vivo to the instrument (20). The end effector (30) may then be pulled from the loader (10) and the latch (55) disengaged from the trigger (54). Tissue is then manipulating by actuating the trigger (54) of the handle (21) to operate the jaws of the end effector (30).

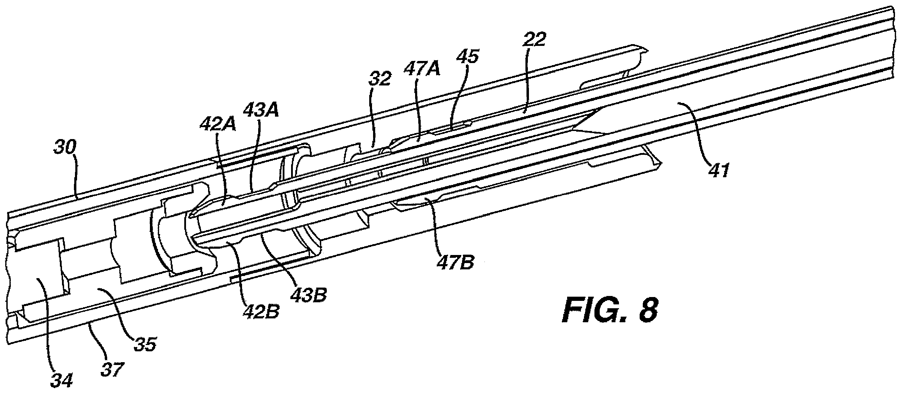

After completing the surgical procedure, the end effector (30) may be detached from the shaft (22). If previously removed, the loader (10) may be reintroduced through the incision (4) into the surgical field. The distal end (32) of the end effector (30) is seated into the distal end (13) of the loader (10), and the pin (41) moved to its unlocked position. The second arms (42) are then proximally withdrawn from the ring (33), deflecting medially as the chamfered portions of the second arms (42) slide over the ring (33) medial surfaces. Accordingly, the device will be in the configuration depicted in Fig. 8. Distally advancing the arms (42) will cause the shaft arms (47) to deflect medially into the opening (48) as the chamfered portions of shaft arms (47) slide over the rib's (32) medial surfaces which simultaneously cause the second arms (42) to deflect medially into the gap (46) facilitating easier separation of the end effector (30) from the shaft (22). The distal advancement of the shaft (22) continutes until the rib (32) unseats from the groove (45). This unseating may be facilitated by the jaws of the end effector (30) being held in a closed position by the tube in the loader distal end (13). The distal end (23) may then be withdrawn from the end effector (30) thus detaching the end effector (30) from the instrument (20). The end effector will be held in the loader (10) by virtue of the engagement feature (16). Removal of the loader (10) from the surgical field will remove the end effector (30). A different end effector may then be attached to the instrument (20), or the instrument (20) may be withdrawn from the surgical field.

Without limitation, the following describe some of the benefits and advantages of the foregoing devices and methods over the prior art. The end effector (30) may have a much larger diameter than the shaft (22); accordingly, the incision (8) can be smaller compared to more traditional laparoscopic instruments resulting in less pain and scarring, and quicker recovery. This also facilitates a small diameter shaft (22) (even less than 3mm), thus potentially eliminating a trocar in the incision (8). The attachment mechanism (40) provides quick end effector (30) exchanges with the instrument (20), thus decreasing surgical time. The loader (10) also facilitates quick end effector (30) exchanges. A kit of multiple end effectors may reduce instrument costs by consolidating a single shaft (22) and handle (21) for all instruments. Many other benefits will be apparent to those skilled in the art.

Having shown and described various embodiments and examples of the present invention, further adaptations of the devices described herein can be accomplished by appropriate modifications by one of ordinary skill in the art without departing from the scope of the appended claims . Several of such potential modifications have been mentioned, and others will be apparent to those skilled in the art. For instance, the specific materials, dimensions, and the scale of drawings will be understood to be non-limiting examples. Accordingly, the scope of the present invention should be considered in terms of the following claims and is understood not to be limited to the details of structure, materials shown and described in the specification and drawings.

## Claims

1. A surgical device (20), comprising:
a) an elongate shaft (22) defining a longitudinal axis, the shaft (22) comprising a distal end and a proximal end;
b) a first arm (47A) on the elongate shaft (22) comprising a mating feature (45), the first arm (47A) being medially deflectable;
c) a second arm (42A) comprising a mating feature (43A) longitudinally spaced from the first arm (47A), the second arm (42A) being axially slideable relative to the elongate shaft (22) and being medially deflectable;
d) an elongate pin (41) positioned medially relative the second arm (42A), the elongate pin (41) being axially slideable relative to the first arm (47A) and the second arm (42A) between a locked position preventing medial deflection of the first arm (47A) and second arm (42A) and an unlocked position where the elongate pin is fully retracted inside the elongate shaft allowing medial deflection of the first arm (47A) and second arm (42A); and
e) a surgical end effector (30) selectively attachable and detachable to the mating feature (43A) of the second arm (42A).

2. The surgical device (20) of claim 1, wherein the mating feature (43A) of the second arm is a lateral notch on the distal end of the first arm, and wherein there is a corresponding mating feature (33) on the surgical end effector (30).

3. The surgical device (20) of claim 2, wherein the mating feature (33) on the surgical end effector comprises a ring dimensioned to mate with the lateral notch (43A).

4. The surgical device (20) of claim 1, comprising two or more second arms (42A, 42B) circumscribing the elongate pin (41).

5. The surgical device (20) of claim 1, comprising two or more first arms (47A, 47B).

6. The surgical device (20) of claim 1, wherein the distal end of the elongate pin (41) comprises an obturator tip.

7. The surgical device (20) of claim 1, further comprising a handle (21) operatively connected to the proximal end of the elongate shaft (22), the handle (21) comprising a trigger (54) controlling the axial movement of the second arm (42A) and an actuator (53) controlling the axial movement of the elongate pin (41).

8. The surgical device (20) of claim 7, wherein the actuator (53) is lockable.

9. The surgical device of claim 7, wherein the end effector (30) has opposable members that move between open and closed positions in response to axial motion of the second arm (42A).

## Patentansprüche

1. Chirurgische Vorrichtung (20), umfassend:
a) einen länglichen Schaft (22), der eine Längsachse definiert, wobei der Schaft (22) ein distales Ende und ein proximales Ende umfasst;
b) einen ersten Arm (47A) an dem länglichen Schaft (22), der ein Eingriffsmerkmal (45) umfasst, wobei der erste Arm (47A) medial auslenkbar ist;
c) einen zweiten Arm (42A), der ein Eingriffsmerkmal (43A) umfasst und in Längsrichtung von dem ersten Arm (47A) beabstandet ist, wobei der zweite Arm (42A) bezogen auf den länglichen Schaft (22) axial verschiebbar und medial auslenkbar ist;
d) einen länglichen Stift (41), der bezogen auf den zweiten Arm (42A) medial positioniert ist, wobei der längliche Stift (41) bezogen auf den ersten Arm (47A) und den zweiten Arm (42A) zwischen einer arretierten Position, die die mediale Auslenkung des ersten Armes (47A) und des zweiten Armes (42A) verhindert, und einer nicht arretierten Position, in der der längliche Stift vollständig ins Innere des länglichen Schafts eingefahren ist, was die mediale Auslenkung des ersten Armes (47A) und des zweiten Armes (42A) erlaubt, axial verschiebbar ist; und
e) ein chirurgisches Greiforgan (30), das wahlweise an dem Eingriffsmerkmal (43A) des zweiten Armes (42A) angebracht und davon gelöst werden kann.

2. Chirurgische Vorrichtung (20) nach Anspruch 1, wobei das Eingriffsmerkmal (43A) des zweiten Armes eine seitliche Aussparung an dem distalen Ende des ersten Armes ist und wobei es ein entsprechendes Eingriffsmerkmal (33) an dem chirurgischen Greiforgan (30) gibt.

3. Chirurgische Vorrichtung (20) nach Anspruch 2, wobei das Eingriffsmerkmal (33) an dem chirurgischen Greiforgan einen Ring umfasst, der bemessen ist, um mit der seitlichen Aussparung (43A) zusammenzupassen.

4. Chirurgische Vorrichtung (20) nach Anspruch 1, umfassend zwei oder mehrere zweite Arme (42A, 42B), die den länglichen Stift (41) umschreiben.

5. Chirurgische Vorrichtung (20) nach Anspruch 1, umfassend zwei oder mehrere erste Arme (47A, 47B).

6. Chirurgische Vorrichtung (20) nach Anspruch 1, wobei das distale Ende des länglichen Stifts (41) eine Obturatorspitze umfasst.

7. Chirurgische Vorrichtung (20) nach Anspruch 1, ferner umfassend einen Griff (21), der funktionell mit dem proximalen Ende des länglichen Schafts (22) verbunden ist, wobei der Griff (21) einen Auslöser (54) umfasst, der die axiale Bewegung des zweiten Armes (42A) steuert, und einen Aktuator (53), der die axiale Bewegung des länglichen Stifts (41) steuert.

8. Chirurgische Vorrichtung (20) nach Anspruch 7, wobei der Aktuator (53) arretierbar ist.

9. Chirurgische Vorrichtung nach Anspruch 7, wobei das Greiforgan (30) gegenüberliegende Elemente aufweist, die sich in Reaktion auf die axiale Bewegung des zweiten Armes (42A) zwischen einer offenen und einer geschlossenen Position bewegen.

## Revendications

1. Dispositif chirurgical (20), comprenant :
a) un arbre allongé (22) définissant un axe longitudinal, l'arbre (22) comprenant une extrémité distale et une extrémité proximale ;
b) un premier bras (47A) sur l'arbre allongé (22) comprenant une caractéristique d'accouplement (45), le premier bras (47A) pouvant être dévié de façon médiale ;
c) un second bras (42A) comprenant une caractéristique d'accouplement (43A) espacée longitudinalement du premier bras (47A), le second bras (42A) pouvant coulisser axialement par rapport à l'arbre allongé (22) et pouvant être dévié de façon médiale ;
d) une broche allongée (41) positionnée de façon médiale par rapport au second bras (42A), la broche allongée (41) pouvant coulisser axialement par rapport au premier bras (47A) et au second bras (42A) entre une position verrouillée empêchant une déviation médiale du premier bras (47A) et du second bras (42A) et une position déverrouillée où la broche allongée est entièrement repliée à l'intérieur de l'arbre allongé permettant une déviation médiale du premier bras (47A) et du second bras (42A) ; et
e) un effecteur terminal chirurgical (30) pouvant être sélectivement fixé à la caractéristique d'accouplement (43A) du second bras (42A) et détaché de celle-ci.

2. Dispositif chirurgical (20) selon la revendication 1, dans lequel la caractéristique d'accouplement (43A) du second bras est une encoche latérale sur l'extrémité distale du premier bras, et dans lequel il existe une caractéristique d'accouplement (33) correspondante sur l'effecteur terminal chirurgical (30).

3. Dispositif chirurgical (20) selon la revendication 2, dans lequel la caractéristique d'accouplement (33) sur l'effecteur terminal chirurgical comprend un anneau dimensionné pour s'accoupler avec l'encoche latérale (43A).

4. Dispositif chirurgical (20) selon la revendication 1, comprenant deux seconds bras (42A, 42B) ou plus circonscrivant la broche allongée (41).

5. Dispositif chirurgical (20) selon la revendication 1, comprenant deux premiers bras (47A, 47B) ou plus.

6. Dispositif chirurgical (20) selon la revendication 1, dans lequel l'extrémité distale de la broche allongée (41) comprend une pointe d'obturateur.

7. Dispositif chirurgical (20) selon la revendication 1, comprenant en outre un manche (21) relié fonctionnellement à l'extrémité proximale de l'arbre allongé (22), le manche (21) comprenant une détente (54) contrôlant le déplacement axial du second bras (42A) et un actionneur (53) contrôlant le déplacement axial de la broche allongée (41).

8. Dispositif chirurgical (20) selon la revendication 7, dans lequel l'actionneur (53) est verrouillable.

9. Dispositif chirurgical selon la revendication 7, dans lequel l'effecteur terminal (30) possède des éléments opposables qui se déplacent entre des positions ouverte et fermée en réponse à un déplacement axial du second bras (42A).
